# FASCICULE DE BREVET EUROPEEN

(11) **EP 0 638 542 B1**
(45) Date de publication et mention de la délivrance du brevet: **21.04.1999**
(21) Numéro de dépôt: 94401804.3
(22) Date de dépôt: 04.08.1994
(51) Int. Cl.: C07C 69/743, A01N 53/00

(54) **Esters pyréthrinoides, dérivés de l'alcool 6-(trifluorométhyl) benzylique, leur procédé de préparation et leur application comme pesticides**
Von 6-Trifluoromethylbenzylalkohol abgeleitete Pyrethrinoidester, Verfahren zu ihrer Herstellung und ihre Anwendung als Pestizide
Pyrethrinoid esters derived from 6-trifluoromethyl benzyl alcohol, their preparation and use as pesticides

(30) Priorité: 05.08.1993 FR 9309653
(43) Date de publication de la demande: 15.02.1995
(73) Titulaire: Hoechst Schering AgrEvo S.A., 75020 Paris (FR)
(72) Inventeur: Babin, Didier, F-78180 Montigny (FR); Benoit, Marc, F-13360 Roquevaire (FR); Bouchet, Raphael, F-93500 Pantin (FR); Demoute, Jean-Pierre, F-93360 Neuilly Plaisance (FR)
(74) Mandataire: Vieillefosse, Jean-Claude

(56) Documents cités:
- EP-A- 0 006 978
- EP-A- 0 010 879
- EP-A- 0 016 513
- EP-A- 0 378 080
- EP-A- 0 551 035
- EP-A- 0 557 192

## Description

La présente invention concerne de nouveaux dérivés de l'alcool 6-trifluorométhyl benzylique, leur procédé de préparation et leur application comme pesticides.

On connaissait des esters d'acides cyclopropane carboxyliques et d'alcool benzylique doués de propriétés pesticides (Cf EP-A-6978, EP-A-10874 et EP-A-16513).

L'invention a pour objet sous toutes les formes stéréoisomères possibles ainsi que leurs mélanges, les composés de formule (I) :

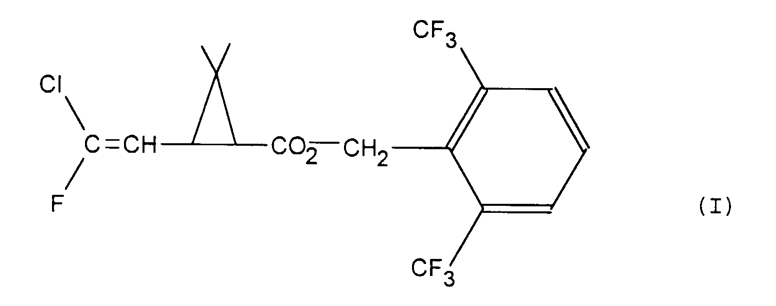

Parmi les composés préférés de l'invention, on peut citer les composés dans lesquels la copule cyclopropanique est de structure 1Rcis.

L'invention a notamment pour objet le produit de l'exemple 1.

L'alcool 2,6-bis (trifluorométhyl) benzylique est un produit nouveau dont la préparation est donnée ci-après dans la partie expérimentale.

Les composés de formule (I) présentent d'intéressantes propriétés qui permettent leur utilisation dans la lutte contre les parasites. Il peut s'agir par exemple de la lutte contre les parasites des végétaux, les parasites des locaux et les parasites des animaux à sang chaud.

C'est ainsi que l'on peut utiliser les produits de l'invention pour lutter contre les insectes, les nématodes et les acariens parasites des végétaux et des animaux.

L'invention a notamment pour objet l'application des composés de formule (I) à la lutte contre les parasites des végétaux, les parasites des locaux et les parasites des animaux à sang chaud.

Les produits de formule (I) peuvent aussi être utilisés pour lutter contre les insectes et autres parasites du sol, par exemple les coléoptères, comme Diabrotica, les taupins et les vers blancs, les myriapodes comme les scutigérelles et les blaniules, et les diptères comme les cécydomies et les lépidoptères comme les noctuelles terricoles.

Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

Les produits de formule (I) sont de plus photostables et sont peu toxiques pour les mammifères.

L'ensemble de ces propriétés fait que les produits de formule (I) correspondent parfaitement aux exigences de l'industrie agrochimique moderne : ils permettent de protéger les récoltes tout en préservant l'environnement.

Les produits de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques de l'espèce de Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique.

L'invention a donc également pour objet les compositions destinées à la lutte contre les parasites des végétaux, les parasites des locaux et les parasites des animaux à sang chaud, caractérisées en ce qu'elles renferment au moins l'un des produits de formule (I) définis ci-dessus et notamment le produit de formule (I) de l'exemple 1.

L'invention a notamment pour objet les compositions insecticides renfermant comme principe actif au moins l'un des produits définis ci-dessus.

L'invention a plus particulièrement pour objet les compositions insecticides définies ci-dessus, destinées à la lutte contre Diabrotica et les autres parasites du sol.

Ces compositions sont préparées selon les procédés usuels de l'industrie agrochimique ou de l'industrie vétérinaire ou de l'industrie des produits destinés à la nutrition animale.

Dans ces compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

L'invention a également pour objet les compositions acaricides et nématicides renfermant comme principe actif au moins un des produits de formule (I) définie ci-dessus.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % en poids de principe actif ou des liquides pour pulvérisation foliaire contenant de 1 à 500 g/l de principe actif. On peut également employer des poudres pour poudrages foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo[2,2-1]5-heptène-2,3-dicarboximide, ou le pipéronyl-bis-2-(2'-n-butoxy éthoxy) éthylacétal (ou tropital).

Les composés de formule (I) présentent une excellente tolérance générale, et l'invention a donc également pour objet les produits de formule (I), pour lutter notamment contre les affections créées par les tiques et les gales chez l'homme et l'animal.

Les produits de l'invention sont notamment utilisés pour lutter contre les poux à titre préventif ou curatif et pour lutter contre la gale.

Les produits de l'invention peuvent être administrés par voie externe, par vaporisation, par shampooing, par bain ou badigeonnage.

Les produits de l'invention à usage vétérinaire peuvent être également administrés par badigeonnage de l'épine dorsale selon la méthode dite méthode "pour-on".

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoides choisi dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcool alpha-cyano 3-phénoxy benzylique des acides chrysanthémiques, par les esters d'alcool 5-benzyl 3-furyl méthylique des acides 2,2-diméthyl 3-(2-oxo 3-tétrahydrothiophénylidène méthyl) cyclopropanecarboxyliques, par les esters d'alcool 3-phénoxy benzylique et d'alcool alpha-cyano 3-phénoxy benzylique des acides 2,2-diméthyl 3-(2,2-dichlorovinyl) cyclopropanecarboxyliques, par les esters d'alcool alpha-cyano 3-phénoxy benzylique d'acides 2,2-diméthyl 3-(2,2-dibromovinyl) cyclopropanecarboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimidométhylique, d'alcool 5-benzyl 3-furyl méthylique, d'alcool 3-phénoxy benzylique et d'alcool alpha-cyano 3-phénoxy benzylique des acides 2,2-diméthyl 3-(1,2,2,2-tétrahaloéthyl) cyclopropanecarboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoides ci-dessus.

L'invention a également pour objet un procédé de préparation des composés de formule (I) caractérisé en ce que l'on soumet un acide de formule (II) : 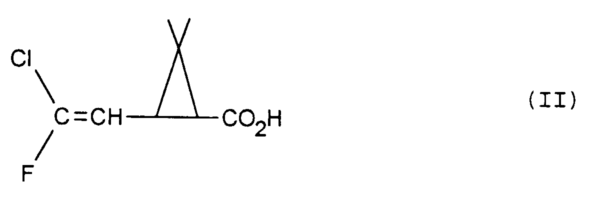 dans laquelle R₁ et R₂ conservent leur signification précédente, ou un dérivé fonctionnel de cet acide, à l'action d'un alcool de formule (III) : 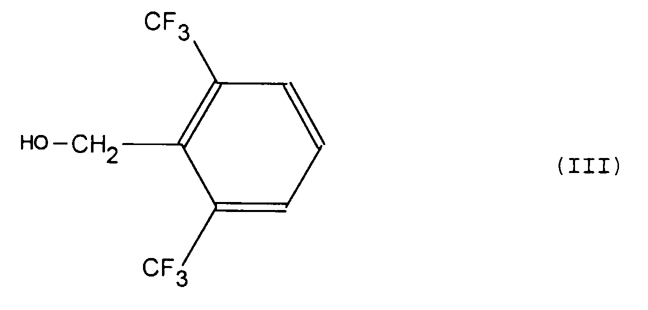 ou d'un dérivé fonctionnel de cet alcool pour obtenir le composé de formule (I) correspondant.

Le dérivé fonctionnel d'acide utilisé est de préférence un chlorure d'acide.

Lorsque l'on fait réagir l'acide de formule (II) sur l'alcool de formule (III), on opère de préférence en présence de dicyclohexylcarbodiimide.

L'alcool 2,6-bis(trifluorométhyl) benzylique est un produit nouveau dont la préparation est donnée ci-après dans la partie expérimentale.

Les acides de formule (II) utilisés comme produits de départ sont des produits connus décrits par exemple dans la demande de brevet européen 0378026.

Les exemples suivants illustrent l'invention.

### Exemple 1 : (1R,cis)-2,2-diméthyl 3-(2-fluoro 2-chloro vinyl) cyclopropanecarboxylate de 2,6-bis(trifluorométhyl)phényl méthyle.

On ajoute goutte à goutte à 0°C dans une solution renfermant 0,4 g d'acide (1R,cis) 2,2-diméthyl 3-(2-fluoro 2-chloro) cyclopropanecarboxylique, 0,49 g d'alcool 2,6-bis(trifluorométhyl) benzylique et 10 ml de chlorure de méthylène, une solution renfermant 0,43 g de dicyclohexylcarbodiimide (DCC), 10 mg de diméthylaminopyridine (DMAP) et 10 ml de chlorure de méthylène. On laisse le mélange réactionnel revenir à la température ambiante. On évapore à sec et chromatographie sur silice en éluant avec le mélange hexane-acétate d'éthyle. On obtient ainsi 0,67 g de produit recherché.

| Analyse : | calculé | trouvé |
|---|---|---|
| C | 48,76 % | 49,0 % |
| H | 3,37 % | 3,3 % |
| Cl | 8,46 % | 8,8 % |
| F | 31,76 % | 31,6 % |

RMN CDCl₃ ppm
5,35 (dd,J=10-29) CH=éthylénique ÞE
5,75 (dd,J=8,5-10) CH=éthylénique ÞZ
2,06(m)
1,81(m) H₃ du cyclopropane
1,19(s)
1,21(s) H des méthyles géminés
1,23(s)
1,75 H₁

### PREPARATION 1 : Alcool 2,6-bis(trifluorométhyl) benzylique

### STADE A : 2,6-bis(trifluorométhyl) benzoate de méthyle.

On agite pendant 30 minutes à 20°C, une solution renfermant 6 g d'acide 2,6-bis(trifluorométhyl) benzoique, 60 ml de tétrahydrofuranne et 11,58 ml d'une solution de soude 2N. On refroidit à 0°C et ajoute 4,26 ml de sulfate de diméthyle. On agite 1 heure à 20°C et ajoute à nouveau 2,1 ml de sulfate de diméthyle et maintient le mélange réactionnel sous agitation pendant 24 heures à 20°C. On le verse sur une solution aqueuse de bicarbonate de sodium, on extrait à l'éther isopropylique puis à l'acétate d'éthyle. On sèche, filtre, rince et amène à sec. On obtient ainsi, après chromatographie sur silice (éluant hexane-acétate d'éthyle (9-1)) 5,59 g du produit recherché.

### STADE B : Alcool 2,6-bis(trifluorométhyl) benzylique.

On ajoute à 0°C, 58 ml d'une solution 1,2 M d'hydrure de diisobutylaluminium (DIBAH) dans une solution renfermant 5,59 g du produit préparé au stade A et 60 ml de toluène. On laisse la température revenir à 20°C et maintient le mélange réactionnel sous agitation pendant 4 heures. On verse dans une solution molaire de tartrate double de potassium et de sodium. On extrait à l'éther isopropylique, sature la phase aqueuse avec du chlorure de sodium. On extrait à l'acétate d'éthyle, sèche, filtre, rince et amène à sec. Après chromatographie sur silice (éluant : hexane-acétate d'éthyle (9-1)) on obtient 4,72 g de produit recherché.

### EXEMPLE 2 : Préparation d'un concentré soluble

On effectue un mélange homogène de :

| | |
|---|---|
| Produit de l'exemple 1 | 0,25 g |
| Butoxyde pipéronyle | 1,00 g |
| Tween 80 | 0,25 g |
| Topanol A | 0,1 g |
| Eau | 98,4 g |

### EXEMPLE 3 : Préparation d'un concentré émulsifiable

On mélange intimement :

| | |
|---|---|
| Produit de l'exemple 1 | 0,015 g |
| Butoxyde de pipéronyle | 0,5 g |
| Topanol A | 0,1 g |
| Tween 80 | 3,5 g |
| Xylène | 95,885 g |

### ETUDE BIOLOGIQUE

### A - Activité sur Diabrotica

Les insectes tests sont des larves de dernier stade de Diabrotica. On traite une rondelle de papier filtre de 9 cm de diamètre, déposée au fond d'une boîte de Pétri, à l'aide de 2 cm³ de solution acétonique du produit à tester. Après séchage on dépose 15 larves par dose et on effectue le contrôle de mortalité 24 heures après le traitement.

Dès la dose de 1 ppm les produits de l'invention présentent une bonne activité.

## Revendications

1. Sous toutes les formes stéréoisomères possibles ainsi que leurs mélanges, le composé de formule (I) : 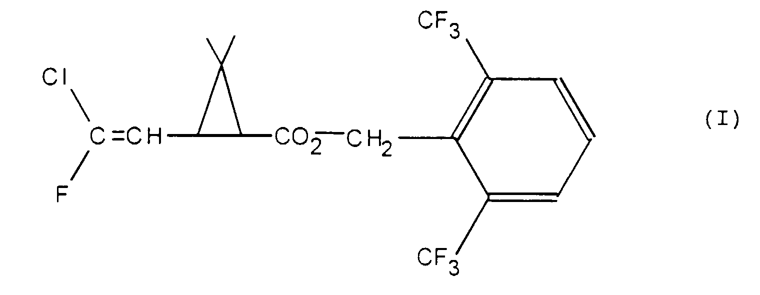 dans lesquels :

2. Les composés de formule (I) tels que définis à la revendication 1 dans lesquels la copule cyclopropanique est de structure 1Rcis.

3. Le composé de formule (I) telle que définie à la revendication 1 dont le nom suit :
le 1Rcis 2,2-diméthyl 3-(2-fluoro 2-chlorovinyl) cyclopropane carboxylate de 2,6-bis(trifluorométhyl) phénylméthyle.

4. Procédé de préparation des composés de formule (I) tels que définis à l'une quelconque des revendications 1 à 3, caractérisé en ce que l'on soumet un acide de formule (II) : 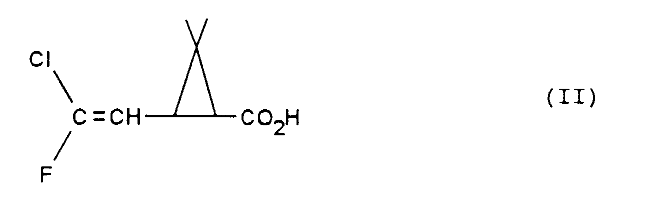 dans laquelle R₁ et R₂ conservent leur signification précédente, ou un dérivé fonctionnel de cet acide, à l'action d'un alcool de formule (III) : 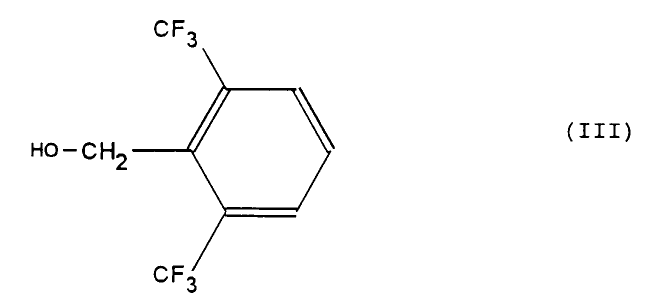 ou d'un dérivé fonctionnel de cet alcool pour obtenir le composé de formule (I) correspondant.

5. Les compositions destinées à la lutte contre les parasites des végétaux, les parasites des locaux et les parasites des animaux à sang chaud, caractérisées en ce qu'elles renferment comme principe actif au moins un composé défini à l'une quelconque des revendications 1 à 3.

6. Les compositions insecticides, caractérisées en ce qu'elles renferment comme principe actif au moins un composé défini à l'une quelconque des revendications 1 à 3.

7. Les compositions insecticides définies à la revendication 5, caractérisées en ce qu'elles sont destinées à la lutte contre DIABROTICA et les autres parasites du sol.

8. Les compositions acaricides et nématicides, caractérisées en ce qu'elles renferment comme principe actif au moins un composé défini à l'une quelconque des revendications 1 à 3.

9. Associations douées d'activité insecticide, acaricide ou nématicide, caractérisées en ce qu'elles contiennent comme matière active, d'une part, un au moins des composés de formule générale (I) et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcool alpha-cyano 3-phénoxy benzylique des acides chrysanthémiques, par les esters d'alcool 5-benzyl 3-furyl méthylique des acides 2,2-diméthyl 3-(2-oxo 3-tétrahydrothiophénylidène méthyl) cyclopropanecarboxyliques, par les esters d'alcool 3-phénoxy benzylique et d'alcool alpha-cyano 3-phénoxy benzylique des acides 2,2-diméthyl 3-(2,2-dichlorovinyl) cyclopropanecarboxyliques, par les esters d'alcool alpha-cyano 3-phénoxy benzylique d'acides 2,2-diméthyl 3-(2,2-dibromovinyl) cyclopropanecarboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimidométhylique, d'alcool 5-benzyl 3-furyl méthylique, d'alcool 3-phénoxy benzylique et d'alcool alpha-cyano 3-phénoxy benzylique des acides 2,2-diméthyl 3-(1,2,2,2-tétrahaloéthyl) cyclopropanecarboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

## Patentansprüche

1. In allen möglichen stereoisomeren Formen sowie ihren Gemischen die Verbindung der Formel (1): 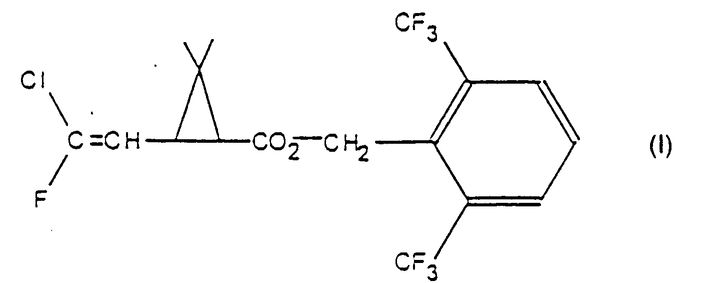 in denen:

2. Die Verbindungen der Formel (1), wie in Anspruch 1 definiert, in denen die Cyclopropankupplung die Struktur 1Rcis besitzt.

3. Die Verbindung der Formel (1), wie in Anspruch 1 definiert, deren Namen folgt:
1Rcis-2,2-D imethyl-3-(2-fluor-2-chlorvinyl )cyclopropancarbonsäure-2,6-bis(trifluormethyl)phenylmethylester.

4. Verfahren zur Herstellung der Verbindungen der Formel (1), wie in einem der Ansprüche 1 bis 3 definiert, dadurch gekennzeichnet, daß man eine Säure der Formel (II): 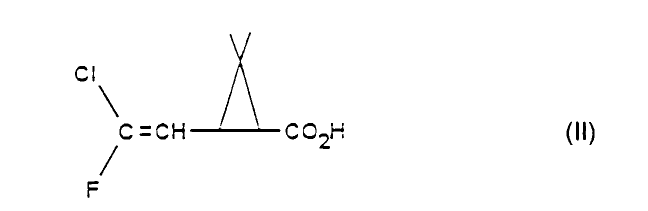 in der R₁ und R₂ ihre vorherige Bedeutung behalten, oder ein funktionelles Derivat dieser Säure der Einwirkung eines Alkohols der Formel (III): 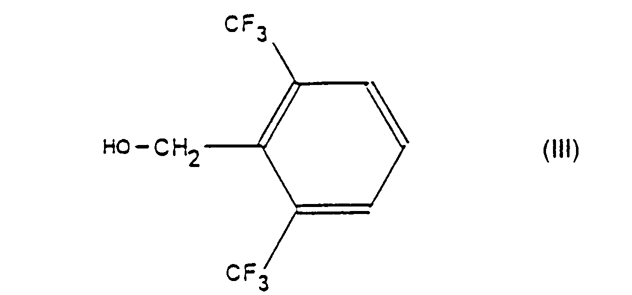 oder eines funktionellen Derivats dieses Alkohols unterwirft, um die entsprechende Verbindung der Formel (I) zu erhalten.

5. Die Zusammensetzungen, die für die Bekämpfung von Pflanzenschädlingen, Raumschädlingen und Parasiten von Warmblütern bestimmt sind, dadurch gekennzeichnet, daß sie als Wirkstoff wenigstens eine Verbindung, die in einem der Ansprüche 1 bis 3 definiert ist, enthalten.

6. Die Insektizid-Zusammensetzungen, dadurch gekennzeichnet, daß sie als Wirkstoff wenigstens eine Verbindung, die in einem der Ansprüche 1 bis 3 definiert ist, enthalten.

7. Die in Anspruch 6 definierten Insektizid-Zusammmensetzungen, dadurch gekennzeichnet, daß sie für die Bekämpfung von DIABROTICA und anderen Bodenschädlingen bestimmt sind.

8. Die Akarizid- und Nematizid-Zusammensetzungen, dadurch gekennzeichnet, daß sie als Wirkstoff wenigstens eine Verbindung, die in einem der Ansprüche 1 bis 3 definiert ist, enthalten.

9. Zusammensetzungen, insektizider, akarizider oder nematizider Wirksamkeit, dadurch gekennzeichnet, daß sie als wirkamen Stoff einerseits wenigstens eine der Verbindungen der allgemeinen Formel (I) und andererseits wenigstens einen der Pyrethrinoidester enthalten, die aus der Gruppe ausgewählt sind, die besteht aus den Estem des Allethrolons, 3,4,5,6-Tetrahydrophthalimidomethylalkohols, 5-Benzyl-3-furyl-methylalkohols, 3-Phenoxy-benzylalkohols und alpha-Cyano-3-phenoxy-benzylalkohols der Chrysanthemumsäuren, aus den Estem des 5-Benzyl-3-furyl-methylalkohols der 2,2-Dimethyl-3-(2-oxo-3-tetrahydrothiophenylidenmethyl)cyclopropansäuren, aus den Estem des 3-Phenoxy-benzylalkohols und alpha-Cyano-3-phenoxy-benzylalkohols der 2,2-Dimethyl-3-(2,2-dichlorvinyl)cyclopropansäuren, aus den Estem des alpha-Cyano-3-phenoxy-benzylalkohols der 2,2-Dimethyl-3-(2,2-dibromvinyl)cyclopropansäuren, aus den Estem des 3-Phenoxy-benzylalkohols der 2-para-Chlorphenyl-2-isopropyl-essigsäuren, aus den Estem des Allethrolons, 3,4,5,6-Tetrahydrophthalimidomethylalkohols, 5-Benzyl-3-furyl-methyl-alkohols, 3-Phenoxy-benzylalkohols und alpha-Cyano-3-phenoxy-benzylalkohols der 2,2-Dimethyl-3-(1,2,2,2-tetrahaloethyl)cyclopropancarbonsäuren, in denen "halo" ein Fluor-, Chlor- oder Bromatom darstellt, wobei es sich versteht, daß die Verbindungen (I) in allen ihren möglichen stereoisomeren Formen sowie den Säure- und Alkoholkupplungen der obigen Pyrethrinoidester existieren können.

## Claims

1. In all the possible stereoisomeric forms as well as their mixtures, the compounds of formula (I) : 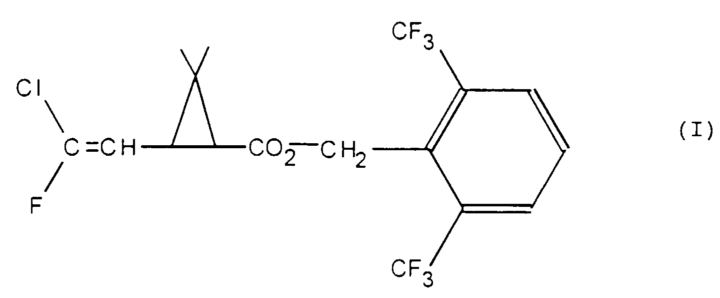 in which:

2. The compounds of formula (I) as defined in claim 1 in which the cyclopropane copula is of 1Rcis structure.

3. The compound of formula (I) as defined in claim 1 the name of which follows:
2,6-bis(trifluoromethyl) phenylmethyl 1Rcis 2,2-dimethyl 3-(2-fluoro 2-chlorovinyl) cyclopropane carboxylate.

4. Preparation process for the compounds of formula (I) as defined in any one of claims 1 to 3, characterized in that an acid of formula (II) : 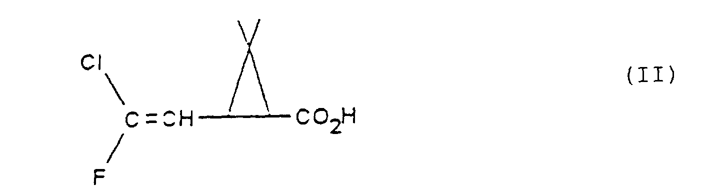 in which R₁ and R₂ retain their previous meaning, or a functional derivative of this acid, is subjected to the action of an alcohol of formula (III): 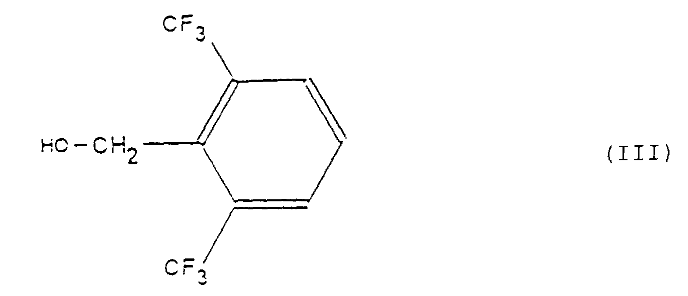 or a functional derivative of this alcohol in order to obtain the corresponding compound of formula (I).

5. The compositions intended for combating parasites of vegetation, parasites of premises and parasites of warmblooded animals, characterized in that they contain at least one compound defined in any one of claims 1 to 3 as active ingredient.

6. The insecticide compositions, characterized in that they contain at least one compound defined in any one of claims 1 to 3 as active ingredient.

7. The insecticide compositions defined in claim 5, characterized in that they are intended to combat DIABROTICA and the other parasites of the soil.

8. The acaricide and nematicide compositions, characterized in that they contain at least one compound defined in any one of claims 1 to 3 as active ingredient.

9. Combinations endowed with insecticide, acaricide or nematicide activity, characterized in that they contain as active ingredient, on the one hand, at least one of the compounds of general formula (I) and on the other hand, at least one of the pyrethrinoid esters chosen from the group constituted by the esters of allethrolone, of 3,4,5,6-tetrahydrophthalimidomethyl alcohol, of 5-benzyl-3-furyl methyl alcohol, of 3-phenoxybenzyl alcohol and of alpha-cyano-3-phenoxybenzyl alcohol with chrysanthemic acids, by the esters of 5-benzyl-3-furyl methyl alcohol with 2,2-dimethyl-3-(2-oxo-3-tetrahydrothiophenylidenemethyl)-cyclopropane-carboxylic acids, by the esters of 3-phenoxybenzyl alcohol and of alpha-cyano-3-phenoxybenzyl alcohol with 2,2-dimethyl-3-(2,2-dichlorovinyl)-cyclopropanecarboxylic acids, by the esters of alpha-cyano-3-phenoxy-benzyl alcohol with 2,2-dimethyl-3-(2,2-dibromovinyl)-cyclopropanecarboxylic acids, by the esters of 3-phenoxybenzyl alcohol with 2-parachlorophenyl-2-isopropyl acetic acids, by the esters of allethrolone, of 3,4,5,6-tetrahydrophthalimidomethyl alcohol, of 5-benzyl-3-furyl methyl alcohol, of 3-phenoxybenzyl alcohol and of alpha-cyano-3-phenoxybenzyl alcohol with 2,2-dimethyl-3-(1,2,2,2-tetrahaloethyl)-cyclopropane-carboxylic acids, in which "halo" represents a fluorine, chlorine or bromine atom, it being understood that the compounds (I) can exist in all their possible stereoisomeric forms as well as the acid and alcohol copulas of the above pyrethrinoid esters.
